(19) Europäisches Patentamt
European Patent Office
Office européen des brevets

(11) Veröffentlichungsnummer: **0 189 788**
**B1**

(12) # EUROPÄISCHE PATENTSCHRIFT

(45) Veröffentlichungstag der Patentschrift: **13.09.89**

(21) Anmeldenummer: **86100537.9**

(22) Anmeldetag: **17.01.86**

(51) Int. Cl.⁴: **A 61 K 45/06**, A 61 K 31/44, A 61 K 31/54, A 61 K 31/62 // (A61K31/44, 31:405, 31:195, 31:19),(A61K31/54, 31:44)

(54) **Synergistische Kombination von Flupirtin und nicht-steroidalen Antiphlogistika.**

(30) Priorität: **23.01.85 DE 3502005**

(43) Veröffentlichungstag der Anmeldung: **06.08.86 Patentblatt 86/32**

(45) Bekanntmachung des Hinweises auf die Patenterteilung: **13.09.89 Patentblatt 89/37**

(84) Benannte Vertragsstaaten: **AT BE CH DE FR GB IT LI LU NL SE**

(56) Entgegenhaltungen:
**EP-A-0 109 036**

**CHEMICAL ABSTRACTS, Band 95, Nr. 21, 23. November 1981, Seite 632, Nr. 187018p, Columbus, Ohio, US; W. VON BEBENBURG et al.: "Synthesis and molecular structure of the structurally novel analgesic Flupirtin", & CHEM.-ZTG. 1981, 105(7-8), 217-19**
**ARZNEIMITTEL FORSCHUNG, DRUG RESEARCH, Band 35(I), Nr. 1, Januar 1985, Seiten 30-43, Edition Cantor, Aulendorf/Württ, DE; V. JAKOVLEV et al.: "Untersuchungen zur pharmakologischen Wirkung von Flupirtin, einem strukturell neuartigen Analgetikum"**

**Die Akte enthält technische Angaben, die nach dem Eingang der Anmeldung eingereicht wurden und die nicht in dieser Patentschrift enthalten sind.**

(73) Patentinhaber: **ASTA Pharma Aktiengesellschaft, Weismüllerstrasse 45, D-6000 Frankfurt am Main 1 (DE)**

(72) Erfinder: **Tibes, Ulrich, Dr., Am Sandberg 102, D-6000 Frankfurt/Main 70 (DE)**
Erfinder: **Weischer, Carl Heinrich, Dr., Schmidtbonnstrasse 8, D-5300 Bonn 1 (DE)**
Erfinder: **Hettche, Helmut, Dr., Buchrainweg 65, D-6050 Offenbach/Main (DE)**
Erfinder: **Breuel, Hans- Peter, Prof., Am Jungstück 34, D-6500 Mainz 43 (DE)**

EP 0 189 788 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Flupirtin ist ein Arzneimittelwirkstoff mit analgetischen Eigenschaften. Sein chemischer Name ist 2-Amino-3-carbethoxyamino-6-(4-fluor-benzylamino)-pyridin mit der folgenden Strukturformel:

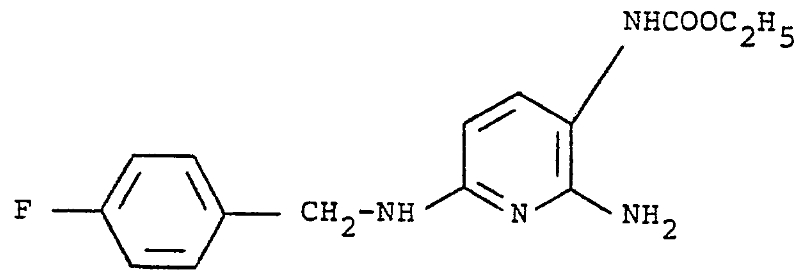

Das Flupirtin und dessen Salze mit physiologisch unbedenklichen Säuren besitzen eine ausgeprägte analgetische Hauptwirkung sowie eine geringe antiphlogistische Wirkungskomponente. Es wurde nun gefunden, daß die Wirkung des Flupirtins und seiner Salze überraschenderweise durch Kombination mit nicht-steroidalen Antiphlogistika synergistisch gesteigert wird, wobei gleichzeitig die Wirkung der Antiphlogistika ebenfalls eine synergistische Steigerung erfährt. Die Wirkstoffe der erfindungsgemäßen Kombination potenzieren sich also gegenseitig in ihrer Wirkung.

Aufgabe der Erfindung ist die Bereitstellung von verbesserten Arzneimitteln mit analgetischer und antiphlogistischer Wirkung.

Die Erfindung betrifft die durch die Patentansprüche definierten Gegenstände.

Die Synergistische Kombination von Flupirtin und nicht-steroidalen Antiphlogistika ist gekennzeichnet durch ein Arzneimittel, enthaltend als Wirkstoff Flupirtin und Acetylsalicylsäure oder ein nicht-steroidales Antiphlogistikum, welches ein Arylessigsäure- oder Arylpropionsäurederivat oder ein Oxicam ist oder ein Salz dieser Verbindungen mit physiologisch unbedenklichen Säuren beziehungsweise physiologisch unbedenklichen Metallen, wobei die Kombination in der Dosierungseinheit 10 bis 1800 mg Flupirtin und 1 bis 1600 mg des Antiphlogistikums enthält und die Menge des nicht-steroidalen Antiphlogistikums 0,05 bis 120 Gewichtsteile bezogen auf einen Gewichtsteil Flupirtin beträgt.

Nach einer bevorzugten Ausführungsform ist das Arzneimittel dadurch gekennzeichnet, daß die Kombination 10 - 1200 mg, vorzugsweise 15 - 900 mg Flupirtin enthält und 2 - 500 mg Acetylsalicylsäure beziehungsweise nicht-steroidales Antiphlogistikum.

In besonders bevorzugter Weise zeichnet sich das erfindungsgemäße Arzneimittel dadurch aus, daß es eine Kombination von 50 - 600 mg, vorzugsweise 50 - 400 mg Flupirtin enthält sowie 5 - 300 mg Acetylsalicylsäure beziehungsweise nicht-steroidales Antiphlogistikum.

Ferner ist Gegenstand der vorliegenden Erfindung ein Verfahren zur Herstellung eines Arzneimittels mit den vorstehend bezeichneten Wirkungen, welches dadurch gekennzeichnet ist, daß man den Wirkstoff Flupirtin und Acetylsalicylsäure oder Flupirtin und ein nicht-steroidales Antiphlogistikum, welches ein Arylessigsäure- oder Arylpropionsäurederivat oder ein Oxicam ist oder ein Salz dieser Verbindungen mit physiologisch unbedenklichen Säuren beziehungsweise physiologisch unbedenklichen Metallen gegebenenfalls mit weiteren üblichen Träger- und/oder Verdünnungs- beziehungsweise Hilfsstoffen zu pharmazeutischen Zubereitungen formuliert, wobei die Menge Acetylsalicylsäure oder nicht-steroidales Antiphlogistikum 0,05 bis 120 Gewichtsteile bezogen auf einen Gewichtsteil Flupirtin beträgt und wobei 10 bis 1800 mg Flupirtin und 1 bis 1600 mg Acetylsalicylsäure oder nicht-steroidales Antiphlogistikum zu einer Dosierungseinheit formuliert werden.

Außerdem wird unterstehend die Verwendung von Flupirtin und Acetylsalicylsäure oder Flupirtin und einem nicht-steroidalen Antiphlogistikum beschrieben, welches ein Arylessigsäure- oder Arylpropionsäurederivat oder ein Oxicam ist oder einem Salz dieser Verbindungen mit physiologisch unbedenklichen Säuren beziehungsweise physiologisch unbedenklichen Metallen, wobei die Kombination in der Dosierungseinheit 10 bis 1800 mg Flupirtin und 1 bis 1600 mg Acetylsalicylsäure oder nicht-steroidales Antiphlogistikum gemäß Anspruch 1 enthält, und die Menge Acetylsalicylsäure oder nicht-steroidales Antiphlogistikum 0,05 bis 120 Gewichtsteile, bezogen auf einen Gewichtsteil Flupirtin, beträgt, zur Herstellung eines analgetisch wirksamen Arzneimittels.

Weiterhin sind die Verfahren zur Herstellung der erfindungsgemäßen Kombination dadurch gekennzeichnet, daß man 1 Gewichtsteil Flupirtin und 0,05 - 120 Gewichtsteile Acetylsalicylsäure oder 1 Gewichtsteil Flupirtin und 0,05 - 120 Gewichtsteile eines nicht-steroidalen Antiphlogistikums, welches ein Arylessigsäure- oder Arylpropionsäurederivat oder ein Oxicam ist oder deren Salze mit physiologisch unbedenklichen Säuren beziehungsweise physiologisch unbedenklichen Metallen zusammen mit üblichen Träger- und/oder Verdünnungs- beziehungsweise Hilfsstoffen bei Temperaturen zwischen 20 und 80°C vermischt beziehungsweise homogenisiert, die so erhaltene Mischung zur Herstellung von Zubereitungen, die in der Dosierungseinheit 1 bis 1800 mg Flupirtin und 1 bis 1600 mg Acetylsalicylsäure oder nicht-steroidales Antiphlogistikum enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpresst.

In einer weiteren Ausführungsform ist das Verfahren zur Herstellung des Arzneimittels dadurch gekennzeichnet, daß man 1 Gewichtsteil Flupirtin und 0,05 - 120 Gewichtsteile Acetylsalicylsäure oder eines nichtsteroidalen Antiphlogistikums gemäß Anspruch 1 oder deren Salze mit physiologisch unbedenklichen Säuren beziehungsweise physiologisch unbedenklichen Metallen, gegebenenfalls mit mindestens einem der Hilfsstoffe Stärke, mikrokristalline Cellulose, Calciumhydrogenphosphat und modifizierte Stärke vermischt, mit einer wässrigen Gelatinelösung oder Stärkelösung oder einem wässrigen Vinylpyrrolidon-Vinylacetat Copolymerisat granuliert und das erhaltene Granulat mit Magnesiumstearat und hochdispersem Siliziumdioxid sowie gegebenenfalls auch Stärke und/oder Cellulose vermischt und zu Tabletten verpresst oder in Kapseln abfüllt.

Schließlich zeichnet sich das Verfahren zur Herstellung der beanspruchten Kombination dadurch aus, daß man 1 Gewichtsteil Flupirtin und 0,05 - 120 Gewichtsteile Acetylsalicylsäure oder eines nicht-steroidalen Antiphlogistikums gemäß Anspruch 1 oder deren Salze mit physiologisch unbedenklichen Säuren beziehungsweise physiologisch unbedenklichen Metallen, gegebenenfalls nach Zusatz von Lecithin in geschmolzenem Hartfett suspendiert und homogenisiert und anschließend die Mischung in Hohlzellen ausgießt.

Die in den Patentansprüchen angegebenen Gewichtsmengen beziehungsweise Gewichtsteile beziehen sich jeweils auf die reinen Wirkstoffe, das heißt nicht auf Salze dieser Wirkstoffe.

Als Antiphlogistika, für die Kombination mit dem Flupirtin werden verwendet das nicht-steroidale Antiphlogistikum Acetylsalicylsäure sowie die nicht-steroidalen Antiphlogistika, welche Arylessigsäure oder Arylpropionsäurederivate oder Oxicame sind, mit ausgeprägter antiphlogistischer Hauptwirkung und einer geringeren analgetischen Nebenwirkungskomponente.

Es handelt sich hierbei um Antiphlogistika, deren Wirkung darauf beruht, daß sie die Bildung von Entzündungsmediatoren (Stoffe, die unter anderem Ödeme auslösen), insbesondere die Bildung der Cyclooxygenaseprodukte, hemmen. Beispielsweise handelt es sich um Essigsäurederivate, die in α-Stellung einen substituierten aromatischen Rest enthalten. Bei diesem aromatischen Rest handelt es sich insbesondere um einen Phenylrest, Indolrest, Indanrest, Indenrest, Pyrrolrest, Thiazolrest, Pyrazolrest, Xanthenrest, Thioxanthenrest oder einen 1,3,4-Trihydro-pyrano[3,4-b]-indolrest. Beispiele für solche nicht-steroidale Antiphlogistika sind: Indometacin, Glucametacin, Sulindac, Zomepirac, Diclofenac, Tolmetin, Amfenac, Fentiazac, Etodolac, Furofenac, Isofezolac, Isoxepac, Tiopinac, Pirazolac. Besonders vorteilhaft ist eine Kombination Flupirtin mit Diclofenac.

Ebenfalls handelt es sich beispielsweise um Propionsäurederivate, die in α- oder β-Stellung wiederum einen substituierten aromatischen Rest enthalten, wobei als aromatischer Rest ein Phenylrest, ein Phenylcarbonylrest, ein Biphenylrest, Naphthylrest, Indolrest, 3H-3-Oxa-indolrest, Thiophenrest, Carbazolrest, Oxazolrest oder ein Furanrest in Frage kommt. Beispiele hierfür sind: Flurbiprofen, Fenoprofen, Ketoprofen, Naproxen, Benoxaprofen, Pirprofen, Indoprofen, Fenbufen. Weiterhin kommen als nicht-steroidale Antiphlogistika Oxicame (Benzothienothiazin-3-carbonsäure-Amide) in Frage, welche an der Carboxyamidgruppe zum Beispiel durch einen Pyridylrest oder einen 5-Methyl-isoxazolylrest substituiert sind. Beispiele hierfür sind: Piroxicam, Isoxicam, Tenoxicam.

Insbesondere handelt es sich bei den Antiphlogistika um Arylessigsäuren beziehungsweise Arylpropionsäuren der Formel

$$\begin{array}{c} R_1 \\ | \\ X - CH - CO_2H \end{array} \qquad I$$

und deren Salze mit physiologisch unbedenklichen Metallen (Kationen) oder Säuren, wobei in der Formel I $R_1$ Wasserstoff ist, falls X einen 2-(2,6-Dichlor-phenylamino)-phenylrest, einen 2-Amino-3-phenylcarbonyl-phenylrest, einen N-Methyl-4-methyl-5-(4-chlor-phenylcarbonyl)-2-pyrrolylrest, einen N-Methyl-5-(4-methyl-phenylcarbonyl)-2-pyrrolylrest, einen 1-(4-Chlor-phenylcarbonyl)-2-methyl-5-methoxy-indolylrest, einen 1-(4-Methylsulfinyl-phenylmethylen)-2-methyl-5-fluor-3-indenylrest, einen 2-Phenyl-4-(4-chlor-phenyl)-5-thiazolylrest, einen 1,3,4-triphenyl-5-pyrazolylrest, einen 2-Ethyl-5-indanylrest, einen 1-(4-Fluor-phenyl)-4-(4-chlorphenyl)-3-pyrazolylrest, einen 9-Oxo-2-xanthenylrest, einen 9-Oxo-3-thioxanthenylrest, einen 1,8-Diethyl-1,3,4-trihydro-pyrano[3,4-b]-1-indolylrest, einen (4,5-Diphenyl-2-oxazolyl)-methylrest, einen (5-Chlor-phenyl-furyl)-hydroxymethylrest oder einen (4-Cyclohexyl-phenyl)-carbonylmethylrest bedeutet, wobei im Falle des 1-(4-Chlor-phenylcarbonyl)-2-methyl-5-methoxy-3-indolylrestes die Carboxygruppe der Verbindung I auch in Form des Glucosamids vorliegen kann und wobei $R_1$ Methyl ist, falls X einen 2-Fluor-4-biphenylrest, einen 3-Phenoxyphenyl-rest, einen 3-Phenylcarbonyl-phenylrest, einen 6-Methoxy-2-naphthylrest, einen [3-Chlor-4-(3-pyrrolino)-phenyl]-rest, einen 4-(1-Oxo-1,3-dihydro-isoindol-2-yl)-phenylrest, einen 6-Chlor-2-carbazolylrest, einen 5-Phenylcarbonyl-2-thienylrest, einen 4-(2-Thienylcarbonyl)-phenylrest oder einen 2-(4-Chlor-phenyl)-5-benzoxazolylrest bedeutet, oder wobei der Strukturteil X-CH($R_1$)- der 2-Acetoxy-phenylrest ist.

Weiterhin handelt es sich bei den erfindungsgemäß zur Verwendung kommenden Antiphlogistika um Oxicame (Benzothiazin-3-carbonsäureamide) der Formel

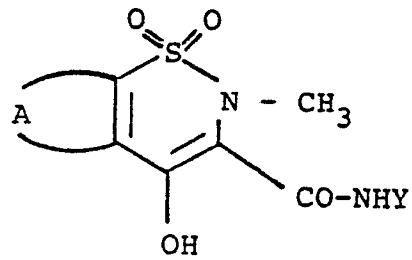

II

und gegebenenfalls deren Salze mit physiologisch unbedenklichen Metallen (Kationen) oder Säuren, wobei A der Formel II ein ankondensierter Benzorest oder ein ankondensierter Thieno-(2,3)-rest ist und Y einen 2-Pyridylrest oder einen 5-Methyl-3-isoxazolylrest darstellt. Ebenfalls kommen als nicht-steroidale Antiphlogistika für die erfindungsgemäße Kombination Fenamate in Frage, wie zum Beispiel Mefanaminsäure, Flufenaminsäure, Meclofenaminsäure (zum Beispiel auch in Form der Salze, zum Beispiel als Natriumsalze).

Das Flupirtin wird vorzugsweise als Säureadditionssalz verwendet, wobei insbesondere die Salze mit Halogenwasserstoffsäuren (zum Beispiel das Hydrochlorid) oder organischen Säuren (zum Beispiel das Maleat oder das Gluconat) in Frage kommen. Die nicht-steroidalen Antiphlogistika werden im allgemeinen nicht in Form ihrer Salze verwendet. Falls sie als Salz eingesetzt werden, handelt es sich beispielsweise um Alkalisalze (zum Beispiel das Natriumsalz).

Die erfindungsgemäße Kombination zeigt beispielsweise im Randall-Selitto-Test, im Essigsäure-Writhing-Test oder im Hot-Plate-Test einen Synergismus der analgetischen Wirkung, die gegenüber der Analgesie des reinen Flupirtin* und dem analgetisch wirksamen Anteil der nicht-steroidalen Antiphlogistika überadditiv gesteigert ist.

Es handelt sich bei der erfindungsgemäßen Kombination um einen funktionellen Synergismus.

Es ist überraschend, daß sowohl die analgetische Hauptwirkung des Flupirtin als auch die analgetisch wirksame Komponente der nicht-steroidalen Antiphlogistika synergistisch gesteigert wird. Ebenso wird überraschend aber auch die antiphlogistische schwache Nebenkomponente des Flupirtins wie auch die antiphlogistische Hauptwirkung der nicht-steroidalen Antiphlogistika synergistisch gesteigert (zum Beispiel im Carrageenin-Ödem-Test, Bradykinin-Ödem-Test oder Adjuvans-Arthritis-Test).

Beispielsweise wird beim Essigsäure-Writhing-Test an der Maus bei einer Dosis von 2,6 mg/kg per os Flupirtin und 0,98 mg/kg per os des Antiphlogistikums Diclofenac die analgetische Wirksamkeit des Flupirtins um den Faktor 17 gesteigert. Die analgetische Wirksamkeit des Diclofenac wird zum Beispiel bei einer Dosis von 2,24 mg/kg Flupirtin und 1,77 mg/kg Diclofenac um den Faktor 7 gesteigert. So wird beispielsweise im Essigsäure-Writhing-Test an der Maus die analgetisch wirksame ED50 des Flupirtins von 44 mg/kg per os auf 2,6 mg/kg per os und die analgetisch wirksame ED50 des Diclofenac von 12 auf 1,8 mg/kg per os gesenkt. (Die Werte beziehen sich auf die zuvor angegebene Mischung.) Hierbei wird Diclofenac bei 0,98 mg/kg per os konstant gehalten und Flupirtin in den Dosen 2,24; 3,57 und 9 mg/kg per os verabfolgt. Im anderen Falle wird Flupirtin bei 2,24 mg/kg konstant gehalten und Diclofenac von 0,49 mg/kg auf 15,6 mg/kg per os jeweils um den Faktor 2 gesteigert. Das zuvor angegebene gilt beispielsweise in gleicher Weise auch für Indometacin, Glucametacin, Naproxen, Piroxicam, Acetylsalicylsäure, Sulindac und Isoxicam. Für die zuletzt genannten Antiphlogistika bewegen sich die Faktoren für die Wirkungssteigerung der analgetischen Wirkung beispielsweise zwischen 2 - 15 gegenüber der analgetischen Nebenwirkungskomponente des reinen Antiphlogistikums (Essigsäure-Writhing-Test der Maus).

* Das Flupirtin wird stets als Maleat, Gluconat oder Hydrochlorid untersucht.

Die Wirkung der erfindungsgemäßen Kombination geht beispielsweise aus folgenden Versuchen hervor: Diese Untersuchungen erfolgten im Essigsäure-Test (Writhing-Test) an der weißen Maus. Hierbei wird sowohl die Flupirtin-Dosis (Gluconat oder Hydrochlorid) konstant gehalten und die Dosis des Antiphlogistikums (Diclofenac) variiert als auch bei konstanter Dosis des Antiphlogistikums die Flupirtin-Dosis geändert und jeweils im ersten Fall die ED50 der analgetischen Komponente des Antiphlogistikums und im zweiten Fall die analgetische Hauptwirkung des Flupirtins in der Kombination bestimmt (Methode nach Forth, Henschler und Rummel, Lehrbuch Pharmakologie und Toxikologie, Ausgabe 1980, Seite 65; Wissenschaftsverlag, Bibliographisches Institut Mannheim, Wien und Zürich).

Die Ergebnisse zeigt die Tabelle 1:

**Tabelle 1**

Writhing-Test nach Koster et al.: Fed. Proc. 18, 412 (1959)
Tier: Weiße Maus (NMRI-Maus); Application: per os

| Wirkstoff-Kombination | Wirkstoffdosis (mg/kg) peroral | Wirkung in % Mittel von 10 Mäusen | ED50 in mg/kg Bestimmung 30 Minuten nach Substanzgabe Methode der linearen Regression |
|---|---|---|---|
| Flupirtin (Gluconat allein | 0,56 | 19,2 | Flupirtin |
| | 1,12 | 28,0 | |
| | 2,24 | 37,6 | |
| | 4,47 | 46,4 | 43,9 |
| | 8,93 | 38,4 | |
| | 17,85 | 22,4 | |
| | 35,7 | 61,6 | |
| Diclofenac allein | 3,9 | 33,3 | Diclofenac |
| | 7,8 | 50,0 | |
| | 15,6 | 45,8 | 12,1 |
| | 31,25 | 66,0 | |
| | 62,5 | 67,4 | |
| | 125,0 | 91,7 | |
| 2,24 mg/kg Flupirtin (Gluconat) + Diclofenac | Diclofenac | | Diclofenac in der Kombination |
| | 0,49 | 31,5 | |
| | 0,98 | 43,4 | |
| | 1,95 | 51,7 | |
| | 3,9 | 55,2 | 1,77 |
| | 7,8 | 76,2 | |
| | 15,6 | 79,7 | |
| 3,57 mg/kg Flupirtin (Hydrochlorid) + Diclofenac | Diclofenac | | Diclofenac in der Kombination |
| | 0,13 | 30,8 | |
| | 0,25 | 35,9 | |
| | 0,49 | 53,2 | 0,56 |
| | 0,98 | 60,9 | |
| | 1,95 | 66,0 | |
| | 3,90 | 71,8 | |
| | 7,80 | 78,8 | |
| 9 mg/kg Flupirtin (Gluconat) + Diclofenac | Diclofenac | | Diclofenac in der Kombination |
| | 0,13 | 41,9 | |
| | 0,25 | 47,3 | |
| | 0,49 | 56,1 | |
| | 0,98 | 62,8 | 0,29 |
| | 1,95 | 66,9 | |
| | 3,90 | 75,0 | |
| | 7,80 | 79,1 | |
| 0,98 mg/kg Diclofenac + Flupirtin (HCl/Gluconat) | | | Flupirtin in der Kombination |
| Flupirtin (Gluconat oder Hydrochlorid) | 2,24 | 43,4 | |
| | 3,57 | 60,9 | 2,6 |
| | 9,0 | 62,8 | |
| 0,49 mg/kg Diclofenac + Flupirtin (HCl/Gluconat) | | | Flupirtin in der Kombination |
| Flupirtin (Gluconat oder Hydrochlorid) | 2,24 | 31,5 | |
| | 3,57 | 53,2 | |
| | 9,00 | 56,1 | 5,1 |

Aus der Tabelle 1 ergibt sich also ein synergistischer Effekt in der analgetischen Wirkung der Kombination

und zwar sowohl hinsichtlich des Flupirtins als auch der analgetischen Nebenwirkungskomponente des Antiphlogistikums Diclofenac.

Die Tabellen 2 und 3 zeigen die Ergebnisse unter Verwendung anderer Antiphlogistika. Hier wird jeweils die Dosis des Flupirtins konstant gehalten und die Dosis des Antiphlogistikums variiert und auf diese Weise jeweils die ED50 der analgetischen Nebenwirkungskomponente des verwendeten Antiphlogistikums ermittelt, wobei diese synergistisch gesteigert wird. Wird hingegen die Flupirtin-Dosis variiert, so findet man in gleicher Weise wie in Tabelle 1 angegeben ist, auch bei den Antiphlogistika der Tabelle 2 die synergistische Steigerung der analgetischen Flupirtin-Wirkung.

**Tabelle 2**

Whriting-Test nach Koster et al.: Fed. Proc. 18, 412 (1959)
Tier: Weiße Maus (NMRI-Maus); Application: per os

| Wirkstoff-Kombination | Wirkstoffdosis (mg/kg) peroral | Wirkung in % Mittel von 10 Mäusen | ED50 in mg/kg Bestimmung 30 Minuten nach Substanzgabe Methode der linearen Regression |
|---|---|---|---|
| Indometacin allein | 2,0 | 17,3 | Indometacin 4,3 |
| | 4,0 | 50,0 | |
| | 8,0 | 86,3 | |
| | 16,0 | 82,1 | |
| 2,24 mg/kg Flupirtin (Gluconat) + Indometacin | Indometacin | | Indometacin in der Kombination |
| | 0,5 | 37,3 | |
| | 1,0 | 39,3 | |
| | 2,0 | 50,0 | 1,4 |
| | 4,0 | 68,0 | |
| | 8,0 | 84,7 | |
| Naproxen allein | 0,38 | 21,7 | Naproxen |
| | 0,75 | 42,0 | |
| | 1,5 | 43,4 | 2,62 |
| | 3,0 | 53,8 | |
| | 6,0 | 70,0 | |
| | 12,0 | 74,1 | |
| | 24,0 | 54,5 | |
| 2,24 mg/kg Flupirtin (Gluoconat) + Naproxen | Naproxen | | Naproxen in der Kombination |
| | 0,10 | 40,1 | |
| | 0,19 | 55,0 | 0,17 |
| | 0,38 | 61,6 | |
| Piroxicam allein | 1,0 | 38,2 | Piroxicam 3,46 |
| | 3,0 | 40,0 | |
| | 4,0 | 53,6 | |
| | 5,0 | 61,8 | |
| | 10,0 | 70,9 | |
| | 20,0 | 49,1 | |
| 2,24 mg/kg Flupirtin (Gluconat) + Pirocixam | Piroxicam | | Piroxicam in der Kombination |
| | 0,5 | 34,5 | |
| | 1,0 | 40,0 | |
| | 3,0 | 58,6 | 1,55 |
| | 4,0 | 64,1 | |
| | 5,0 | 72,4 | |
| | 10,0 | 80,0 | |
| Acetylsalicylsäure allein | 25,0 | 11,0 | Acetylsalicylsäure 148,07 |
| | 50,0 | 30,1 | |
| | 75,0 | 39,0 | |
| | 100,0 | 41,1 | |
| | 150,0 | 43,2 | |
| | 200,0 | 59,6 | |
| | 400,0 | 71,2 | |

| | | | |
|---|---|---|---|
| 2,24 mg/kg Flupirtin (Gluconat) + Acetylsalicylsäure (ASS) | ASS | | Acetylsäure in der Kombination |
| | 12,50 | 39,4 | |
| | 25,0 | 41,8 | |
| | 50,0 | 55,8 | 31,20 |
| | 100,0 | 68,5 | |
| | 200,0 | 83,6 | |
| | 400,0 | 89,1 | |
| Sulindac allein | 0,47 | 29,6 | Sulindac |
| | 0,94 | 44,6 | |
| | 1,88 | 49,7 | |
| | 3,75 | 56,0 | 2,3 |
| | 7,50 | 61,0 | |
| | 15,00 | 71,1 | |
| | 30,00 | 72,3 | |
| 2,24 mg/kg Flupirtin (Hydrochlorid) + Sulindac | Sulindac | | Sulindac in der Kombination |
| | 0,06 | 39,5 | |
| | 0,12 | 44,6 | |
| | 0,24 | 58,0 | 0,15 |
| | 0,47 | 66,2 | |
| | 0,94 | 72,6 | |
| Isoxicam allein | 3,0 | 10,1 | Isoxicam |
| | 4,0 | 30,2 | |
| | 5,0 | 36,7 | |
| | 6,0 | 46,0 | 6,6 |
| | 7,0 | 51,1 | |
| | 8,0 | 62,6 | |
| | 10,0 | 66,2 | |
| 2,24 mg/kg Flupirtin (Hydrochlorid) + Isoxicam | Isoxicam | | Isoxicam in der Kombination |
| | 2,0 | 42,3 | |
| | 4,0 | 44,7 | |
| | 5,0 | 50,0 | 4,2 |
| | 6,0 | 52,0 | |
| | 7,0 | 54,0 | |
| | 8,0 | 66,7 | |
| | 10,0 | 70,0 | |

**Tabelle 3**

Randall-Selitto-Test nach Randall, Selitto, Arch. int. Pharmacodyn. 111, Seite 409 (1957)
Tier: Albano-Ratte (Sprague-Dawley); Application: per os

| Wirkstoff-Kombination | Wirkstoffdosis (mg/kg) peroral | Wirkung in % Mittel von 6 Ratten | ED50 in mg/kg Bestimmung 30 Minuten nach Substanzgabe Methode der linearen Regression |
|---|---|---|---|
| Flupirtin (Hydrochlorid) allein | 10,0 | - 8,3 | Flupirtin |
| | 20,0 | 23,2 | |
| | 30,0 | 45,8 | 30,94 |
| | 40,0 | 67,5 | |
| Diclofenac (allein) | 1,0 | 20,99 | Diclofenac |
| | 3,0 | 32,60 | |
| | 10,0 | 100,55 | 2,95 |
| | 30,0 | 135,36 | |
| 30,0 mg/kg Flupirtin (Hydrochlorid) + Diclofenac | Diclofenac | | Diclofenac in der Kombination |
| | 0,5 | 28,57 | |
| | 1,0 | 55,71 | 1,11 |
| | 3,0 | 63,33 | |

| | | | |
|---|---|---|---|
| | 10,0 | 107,14 | |
| Indometacin allein | 1,0<br>2,0<br>4,0<br>8,0<br>16,0 | 12,3<br>22,05<br>58,97<br>114,87<br>127,18 | Indometacin 2,77 |
| 30,0 mg/kg Flupirtin (Hydrochlorid) + Indometacin | Indometacin<br>0,05<br>0,1<br>1,0 | 28,4<br>76,9<br>91,12 | Indometacin in der Kombination<br>0,069 |

Die niedrigste, bereits analgetisch wirksame Dosis im Writhing-Test an der Maus liegt beispielsweise bei 2,24 mg/kg per os Flupirtin allein und 3,9 mg/kg per os Diclofenac allein vor. Die niedrigste, bereits analgetisch wirksame Dosis im Writhing-Test an der Maus liegt für die Kombination beispielsweise bei 2,24 mg/kg per os Flupirtin und 0,49 mg/kg per os Diclofenac vor.

Beispielsweise findet man am gleichen Tiermodell eine deutlich erkennbare analgetische Wirkung bereits bei folgenden Dosen für die Kombination von:

2,24 mg/kg per os Flupirtin und 0,5 mg/kg per os Indometacin oder
2,24 mg/kg per os Flupirtin und 0,1 mg/kg per os Naproxen oder
2,24 mg/kg per os Flupirtin und 0,5 mg/kg per os Piroxicam oder
2,24 mg/kg per os Flupirtin und 0,06 mg/kg per os Sulindac oder
2,24 mg/kg per os Flupirtin und 2,0 mg/kg per os Isoxicam oder
2,24 mg/kg per os Flupirtin und 12,5 mg/kg per os Acetylsalicylsäure.

Als allgemeine Dosisbereiche für die analgetische Wirkung im Writhing-Test an der Maus kommen beispielsweise in Frage:

2,24 mg/kg per os Flupirtin und 0,49 - 15 mg/kg per os Diclofenac oder
2,24 mg/kg per os Flupirtin und 0,5 - 8 mg/kg per os Indometacin oder
2,24 mg/kg per os Flupirtin und 0,1 - 12 mg/kg per os Naproxen oder
2,24 mg/kg per os Flupirtin und 0,5 - 10 mg/kg per os Piroxicam oder
2,24 mg/kg per os Flupirtin und 0,06 - 0,94 mg/kg per os Sulindac oder
2,24 mg/kg per os Flupirtin und 2,0 - 10 mg/kg per os Isoxicam oder
2,24 mg/kg per os Flupirtin und 12,5 - 400 mg/kg per os Acetylsalicylsäure.

In dem Writhing-Test an der Maus kann das Gewichtsverhältnis von Flupirtin zu dem Antiphlogistikum Diclofenac beispielsweise sein:

1 Gewichtsteil Flupirtin auf von 0,01 bis 200 Gewichtsteile Antiphlogistikum, vorzugsweise 1 Gewichtsteil Flupirtin auf 0,01 bis 100 Gewichtsteile Antiphlogistikum, insbesondere 1 Gewichtsteil Flupirtin auf 0,01 bis 50 Gewichtsteile Antiphlogistikum.

Die Gewichtsverhältnisse von Flupirtin zu dem Antiphlogistikum für die erfindungsgemäße Kombination können im Writhing-Test an der Maus beispielsweise sein:

| | |
|---|---|
| Flupirtin: Diclofenac | von 1 : 0,01 bis 1 : 7,0 |
| Flupirtin: Indometacin | von 1 : 0,06 bis 1 : 3,57 |
| Flupirtin: Naproxen | von 1 : 0,17 bis 1 : 5,36 |
| Flupirtin: Piroxicam | von 1 : 0,22 bis 1 : 4,46 |
| Flupirtin: Sulindac | von 1 : 0,027 bis 1 : 1,67 |
| Flupirtin: Indometacin | von 1 : 0,89 bis 1 : 4,46 |
| Flupirtin: Acetylsalicylsäure | von 1 : 5,58 bis 1 : 178,6 |

Die Gesamtdosis für die Kombination in den Tierversuchen liegt beispielsweise zwischen 1 mg/kg und 300 mg/kg, vorzugsweise zwischen 2 und 100 mg/kg insbesondere zwischen 2 und 50 mg/kg per os. Beispielsweise erhält man für die synergistische analgetische Wirkung von Flupirtin und Antiphlogistikum im Writhing-Test an der Maus eine 50 %-ige Schmerzhemmung bei folgenden Dosen:

9,0 mg/kg per os Flupirtin und 0,29 mg/kg per os Diclofenac oder
2,24 mg/kg per os Flupirtin und 1,4 mg/kg per os Indometacin oder
2,24 mg/kg per os Flupirtin und 0,17 mg/kg per os Naproxen oder
2,24 mg/kg per os Flupirtin und 1,55 mg/kg per os Piroxicam oder
2,24 mg/kg per os Flupirtin und 4,2 mg/kg per os Isoxicam oder
2,24 mg/kg per os Flupirtin und 0,15 mg/kg per os Sulindac oder

2,24 mg/kg per os Flupirtin und 31,2 mg/kg per os Acetylsalicylsäure.

In Bezug auf die antiphlogistische Wirkung wird beispielsweise im Bradykinin-Ödem-Test mit einer Kombination 30 mg/kg Flupirtin und 9 mg/kg per os Diclofenac die antiphlogistische Wirkung des Flupirtins um den Faktor 2 und jene des Diclofenac um mindestens den Faktor 2 gesteigert (Adjuvans-Arthritis-Test, Carrageenin-Ödem-Test, Bradykinin-Ödem-Test an der Ratte; die synergistische Wirkungssteigerung an der Ratte ist besonders ausgeprägt, wenn in der Kombination Flupirtin/Diclofenac mindestens 30 mg Flupirtin vorliegen). Siehe hierzu die folgende Tabelle:

Bradykinin-Ödem-Test in Anlehnung und Modifikation der Methode von Mörsdorf et al (Arch. int. Pharmacodyn. 192, 111 - 127 (1971). Tier: Albinoratte (Sprague-Dawley);
Applikation: per os

| Wirkstoffkombination | Wirkstoffdosis (mg/kg) peroral | Wirkung in % - = Hemmung) Mittel von 6 Ratten | $ED_{50}$ in mg/kg Methode der linearen Regression |
|---|---|---|---|
| Flupirtin (Hydrochlorid) allein | 5,0 | + 4,6 | Flupirtin keine $ED_{50}$ bestimmbar |
| | 10,0 | −25,0 | |
| | 20,0 | −13,6 | |
| | 30,0 | − 2,3 | |
| Diclofenac allein | 1,0 | + 2,9 | Diclofenac keine $ED_{50}$ bestimmbar |
| | 3,0 | + 8,6 | |
| | 9,0 | −31,4 | |
| | 27,0 | −45,7 | |
| 30,0 mg/kg Flupirtin (Hydrochlorid) + Diclofenac | Diclofenac | | Diclofenac in der Kombination 4,8 |
| | 1,0 | −16,4 | |
| | 3,0 | −32,8 | |
| | 9,0 | −67,2 | |

Für die erfindungsgemäßen Kombinationen kommen zum Beispiel folgende Indikationen in Betracht: Entzündliche, degenerative artikuläre und extraartikuläre rheumatische Erkrankungen, Morbus Bechterew, nichtrheumatische Entzündungs- und Schwellungszustände, nichtrheumatische Schmerzzustände, chronische Polyarthritis, Arthrosis deformans, Weichteilrheumatismus, postoperative Schmerzen, insbesondere Schmerzen nach Hals-, Nasen- und Ohren-Eingriffen, Zahnextraktionen und unfallchirurgischen Eingriffen; Schmerzen nach Traumen, insbesondere nach Frakturen, Luxationen und Distorsionen; Schmerzen bei entzündlichen Zuständen im Genitalbereich, insbesondere bei Endometritis, Adnexitis und Pelveoperitonitis; Carzinom-Schmerzen; Dysmenorrhoe. Kontraindikationen sind beispielsweise: Gastrointestinale Beschwerden, Magen- und Darmulzera, schwer eingeschränkte Nieren- und Leberfunktion, Asthma.

Die Tagesdosen der erfindungsgemäßen Kombination bestehen beispielsweise aus 100 bis 1200 mg, vorzugsweise 100 bis 800 mg, insbesondere 100 bis 600 mg oder auch 100 bis 300 mg Flupirtin und 1 - 1600 mg beziehungsweise 2 - 1200 oder 5 - 1000 mg, vorzugsweise 10 - 600 mg, insbesondere 15 - 300 mg des nicht-steroidalen Antiphlogistikums (bei Verwendung von Acetylsalicylsäure kann diese auch als Tagesdosis bis zu 3000 mg vorliegen). Bei Carcinom-Schmerzen kann die Flupirtinmenge insbesondere auch zwischen 1000 - 1500 mg, vorzugsweise 1000 - 1200 mg liegen. Die Tagesdosen können in Form einer einmaligen Verabreichung der gesamten Menge oder in Form von 1 bis 6, insbesondere 1 - 4 Teildosen pro Tag eingesetzt werden. Im allgemeinen ist eine Verabreichung 1 bis 4mal, insbesondere 1 - 3mal täglich bevorzugt.

Die Dosierungseinheit der Kombination aus Flupirtin und dem nicht-steroidalen Antiphlogistikum enthält 10 - 1800 mg Flupirtin und 1 - 1600 mg nicht-steroidales Antiphlogistikum. Im allgemeinen verwendet man 10 bis 300 mg, vorzugsweise 15 bis 200 mg, insbesondere 25 bis 150 mg Flupirtin und 1 bis 400 mg vorzugsweise 1 bis 350 mg, insbesondere 1 bis 300 mg nicht-steroidales Antiphlogistikum, wobei diese Dosierungseinheit zum Beispiel 1 bis 6mal, insbesondere 2 bis 4mal täglich verabreicht werden kann.

Selbstverständlich können auch galenische Zubereitungen hergestellt werden, welche die oben angegebene Dosierungseinheit 2- bis beispielsweise 6mal enthalten. So können beispielsweise Tabletten oder Kapseln der erfindungsgemäßen Kombination hergestellt werden, die 25 - 900 mg der Flupirtin-Komponente enthalten (bei Sachets und Granulaten zum Beispiel 25 - 1800 mg Flupirtin).

Im Falle der nicht-steroidalen Antiphlogistika vom Typ der "Arylpropionsäuren" liegt die Tagesdosis der antiphlogistisch wirksamen Komponente zum Beispiel zwischen 50 - 1500 mg. Im allgemeinen gelten hinsichtlich der antiphlogistischen Komponenten beispielsweise die hierfür bekannten und vorgeschlagenen Tagesdosen. Derartige Dosierungen sind beispielsweise die folgenden:

| nicht-steroidales | Tagesdosen | Einzeldosen | Applikations- |
|---|---|---|---|

| Antiphlogistikum | | | häufigkeit |
|---|---|---|---|
| Diclofenac | 100 mg | 50 - 100 mg | 1 - 3 mal |
| Indometacin | bis maximal 200 mg | 25 - 100 mg | 1 - 6 mal |
| Naproxen | 500 - 1000 mg | 250 - 500 mg | 2 - 4 mal |
| Sulindac | 400 mg | 100 mg | 2 x täglich 2 Tabletten |
| Isoxicam | 200 - 300 mg | 100 - 300 mg | 1 mal |
| Acetylsalicylsäure | bis maximal 5000 mg, insbesondere 500 mg | 500 - 1000 mg | 1 - 8 mal, insbesondere 1 - 3 mal |
| Piroxicam | bis maximal 40 mg, insbesondere 20 mg | 20 mg | 1 mal |

Für die Kombination von Flupirtin und Diclofenac liegt die Tagesdosis im allgemeinen bei 100 - 1500 mg, vorzugsweise 100 - 1200 mg, insbesondere 100 - 800 mg Flupirtin und 10 - 200 mg, insbesondere 10 - 120 mg Diclofenac 1 mal täglich. Insbesondere beträgt diese Tagesdosis etwa 100 - 1000 mg Flupirtin und etwa 10 - 150 mg Diclofenac einmal täglich. Bei mehreren Applikationen täglich sind die genannten Dosen entsprechend zu teilen.

Die Dosierungseinheit der Kombination aus Flupirtin und Diclofenac enthält beispielsweise 10 - 300 mg, vorzugsweise 15 - 200 mg, insbesondere 25 - 150 mg Flupirtin und 3 - 50 mg, vorzugsweise 5 - 30 mg, insbesondere 8 - 20 mg Diclofenac, wobei diese Dosierungseinheit 1 - 6mal täglich verabreicht werden kann. Eine günstige Dosierungseinheit enthält zum Beispiel ca. 100 mg Flupirtin und ca. 25 mg Diclofenac, die zum Beispiel 3mal täglich verabreicht wird.

Im folgenden sind beispielhaft für die Kombination von Flupirtin mit anderen Antiphlogistika die in einer Dosierungseinheit vorkommenden Mengen des jeweiligen Antiphlogistikums aufgeführt, wobei für das Flupirtin jeweils die Bereiche in Frage kommen, die beispielsweise oben für die Kombination mit Diclofenac angegeben sind:

Indometacin (Menge Indometacin in der Kombination mit Flupirtin pro Dosierungseinheit):
5 bis 100 mg, vorzugsweise 10 bis 70 mg, insbesondere 15 bis 50 mg.

Naproxen (Menge Naproxen in der Kombination mit Flupirtin pro Dosierungseinheit):
25 bis 250 mg, vorzugsweise 50 bis 125 mg, insbesondere 70 bis 100 mg.

Sulindac (Menge Sulindac in der Kombination mit Flupirtin pro Dosierungseinheit):
2 bis 100 mg, vorzugsweise 5 bis 75 mg, insbesondere 10 bis 50 mg.

Isoxicam (Menge Isoxicam in der Kombination mit Flupirtin pro Dosierungseinheit):
10 bis 150 mg, vorzugsweise 20 bis 100 mg, insbesondere 30 bis 75 mg.

Piroxicam (Menge Piroxicam in der Kombination mit Flupirtin pro Dosierungseinheit):
1 bis 10 mg, vorzugsweise 1 bis 7,5 mg, insbesondere 2 bis 5 mg.

Acetylsalicylsäure (ASS) (Menge Acetylsalicylsäure in der Kombination mit Flupirtin pro Dosierungseinheit):
100 bis 1500 mg, vorzugsweise 200 bis 1000 mg, insbesondere 300 bis 500 mg.

Auch hinsichtlich dieser Dosierungseinheiten gilt zum Beispiel das bereits zuvor gesagte, daß selbstverständlich auch solche galenischen Zubereitungen hergestellt werden können, die die in der angegebenen Dosierungseinheit enthaltenen Wirkstoffmengen 2- bis beispielsweise 6mal enthalten.

Vorzugsweise wird das Arzneimittel oral verabreicht. Flupirtin und das jeweilige Antiphlogistikum können in jeweils getrennten Formulierungen oder zusammen in einer galenischen Formulierung verwendet werden. Entsprechend einer bevorzugten Ausführung der Erfindung können die Arzneimittel in Form einer einzigen Dosis (das heißt in Form einer Mischung zur peroralen, parenteralen (intravenösen, intramuskulären, subcutanen) rektalen, transdermalen oder vaginalen Verabreichung) formuliert werden, beispielsweise in Form von Tabletten, Kapseln, Pillen, Dragees, Granulaten, Suppositorien, Pellets, Pflaster, einer Lösung, Suspension

oder Emulsion, wobei die Wirkstoffe mit entsprechenden Hilfs- und Trägerstoffen kombiniert werden.

Das nicht-steroidale Antiphlogistikum liegt in der Kombination bezogen auf einen Gewichtsteil Flupirtin in folgendem Verhältnis vor: 0,05 bis 120 Gewichtsteile. Vorzugsweise verwendet man 0,05 bis 60 Gewichtsteile Antiphlogistikum.

Falls man Acetylsalicylsäure ausnimmt, enthält die Kombination zum Beispiel auf einen Gewichtsteil Flupirtin 0,05 bis 40, vorzugsweise 0,1 bis 20 Gewichtsteile Antiphlogistikum. Speziell bei der Kombination von Flupirtin mit Acetylsalicylsäure enthält die Kombination auf einen Gewichtsteil Flupirtin beispielsweise 10 bis 120, vorzugsweise 15 bis 60, insbesondere 20 bis 30 Gewichtsteile Acetylsalicylsäure.

Im allgemeinen werden für die Kombinationen mit den nicht-steroidalen Antiphlogistika vom Typ der Arylessigsäuren beziehungsweise Arylpropionsäuren beispielsweise 0,1 bis 25 mg nicht-steroidales Antiphlogistikum und 5 - 150 mg Flupirtin, vorzugsweise 0,2 - 20 mg nicht-steroidales Antiphlogistikum und 10 - 100 mg Flupirtin, insbesondere 0,5 - 15 mg nicht-steroidales Antiphlogistikum und 20 - 60 mg Flupirtin zum Arzneimittel formuliert. Für die Kombinationen mit den nicht-steroidalen Antiphlogistika vom Typ der Oxicame werden beispielsweise 0,1 - 20 mg nicht-steroidales Antiphlogistikum und 5 - 150 mg Flupirtin, vorzugsweise 0,2 - 15 mg nicht-steroidales Antiphlogistikum und 10 - 100 mg Flupirtin, insbesondere 0,4 - 8 mg nicht-steroidales Antiphlogistikum und 20 - 60 mg Flupirtin zum Arzneimittel formuliert. Diese zuvor angegebenen Gewichtsmengen gelten vorzugsweise für homogene Mischungen von nicht-steroidalen Antiphlogistika und Flupirtin (zum Beispiel Einschichttablette). Andere Mengen und Mengenverhältnisse sind selbstverständlich ebenfalls möglich, insbesondere auch bei Kapseln oder Zweischichttabletten.

Es können beispielsweise Tabletten verschiedener Größe hergestellt werden, beispielsweise mit einem Gesamtgewicht von etwa 50 - 800 mg. Sie enthalten die Wirkstoffe in den vorgenannten Mengen und übliche Trägerstoffe und/oder Verdünnungsmittel und/oder Hilfsstoffe. Diese Tabletten können auch zur Verabreichung von Teildosen vorgesehen sein. In entsprechender Weise können beispielsweise auch andere Zubereitungen, wie zum Beispiel Gelatinekapseln oder Retardformen formuliert werden.

Flüssige Arzneimittelpräparate können durch Lösen oder Suspendieren der erfindungsgemäßen Kombination der Wirkstoffe in üblichen flüssigen Trägern hergestellt werden, wobei beispielsweise Dosen eingestellt werden, die der Menge von 1 - 3 Teelöffel entsprechen.

Derartige Formulierungen können den Patienten zum Beispiel in 1 - 4 Dosen pro Tag verabreicht werden.

Die Dosierungseinheit der erfindungsgemäßen Kombination kann beispielsweise enthalten:

a) bei peroralen Arzneiformen:
Flupirtin: 10 bis 300 mg, vorzugsweise 15 bis 200 mg, insbesondere 25 bis 150 mg;
Antiphlogistika der Formel I: beispielsweise 1 bis 400 mg, vorzugsweise 1 bis 350 mg, insbesondere 1 bis 300 mg.
Bei einer Kombination mit Acetylsalicylsäure enthält die Dosierungseinheit zum Beispiel 100 bis 1500 mg, vorzugsweise 200 bis 1000 mg, insbesondere 300 bis 500 mg Acetylsalicylsäure.
Bei einer Kombination mit Oxicamen enthält die orale Dosierungseinheit beispielsweise 1 bis 10 mg, vorzugsweise 1 bis 7,5 mg, insbesondere 2 bis 5 mg des Oxicams.
(Diese Dosen können beispielsweise 1 - 6, vorzugsweise 1 - 4, insbesondere 1 - 3mal täglich verabreicht werden.)

b) bei parenteralen Arzneiformen (zum Beispiel intravenös, intramuskulär):
Flupirtin: 50 bis 200 mg, vorzugsweise 50 bis 150 mg, insbesondere 100 bis 120 mg;
Antiphlogistika der Formel I: beispielsweise 1 bis 400 mg, vorzugsweise 1 bis 350 mg, insbesondere 1 bis 300 mg.
Bei einer Kombination mit Acetylsalicylsäure enthält die Dosierungseinheit zum Beispiel 100 bis 1500 mg, vorzugsweise 200 bis 1000 mg, insbesondere 300 bis 500 mg Acetylsalicylsäure.
Bei einer Kombination mit Oxicamen enthält die parenterale Dosierungseinheit beispielsweise 1 bis 10 mg, vorzugsweise 1 bis 7,5 mg, insbesondere 2 bis 5 mg des Oxicams.
(Diese Dosen können beispielsweise 1 - 6, vorzugsweise 1 - 4, insbesondere 1 - 3mal täglich verabreicht werden.)

c) bei Arzneiformen zur rektalen oder vaginalen Applikation:
Flupirtin: 75 bis 450 mg, vorzugsweise 100 bis 350 mg, insbesondere 150 bis 300 mg;
Antiphlogistika der Formel I: beispielsweise 1 bis 400 mg, vorzugsweise 1 bis 350 mg, insbesondere 1 bis 300 mg.
Bei einer Kombination mit Acetylsalicylsäure enthält die Dosierungseinheit zum Beispiel 100 bis 1500 mg, vorzugsweise 200 bis 1000 mg, insbesondere 300 bis 500 mg Acetylsalicylsäure.
Bei einer Kombination mit Oxicamen enthält die orale Dosierungseinheit beispielsweise 1 bis 10 mg, vorzugsweise 1 bis 7,5 mg, insbesondere 2 bis 5 mg des Oxicams.
(Diese Dosen können beispielsweise 1 - 6, vorzugsweise 1 - 4, insbesondere 1 bis 3mal täglich verabreicht werden.)

d) bei Arzneiformen zur Applikation auf die Haut und Schleimhäute (zum Beispiel als Lösungen, Lotionen, Emulsionen, Salben, Pflaster und so weiter):
Flupirtin: 10 bis 300 mg, vorzugsweise 15 bis 200 mg, insbesondere 25 bis 150 mg;
Antiphlogistika der Formel I: beispielsweise 1 bis 400 mg, vorzugsweise 1 bis 350 mg, insbesondere 1 bis 300 mg.

Bei einer Kombination mit Acetylsalicylsäure enthält die Dosierungseinheit zum Beispiel 100 bis 1500 mg, vorzugsweise 200 bis 1000 mg, insbesondere 300 bis 500 mg Acetylsalicylsäure.
Bei einer Kombination mit Oxicamen enthält eine solche Dosierungseinheit beispielsweise 1 bis 10 mg, vorzugsweise 1 bis 7,5 mg, insbesondere 2 bis 5 mg des Oxicams.
(Diese Dosen können beispielsweise 1 - 6, vorzugsweise 1 - 4, insbesondere 1 - 3mal täglich verabreicht werden.)

Die in den vorangegangenen Seiten erwähnten Mengenbereiche für die Dosierungseinheiten von Flupirtin und dem jeweiligen Antiphlogistikum sind gegeneinander austauschbar. So kann beispielsweise bei der Kombination Flupirtin - Diclofenac die Dosierungseinheit 10 - 300 mg Flupirtin und 3 - 50 mg Diclofenac oder 10 - 300 mg Flupirtin und 8 - 20 mg Diclofenac oder 25 - 150 mg Flupirtin und 3 - 50 mg Diclofenac oder 25 - 150 mg Flupirtin und 5 - 30 mg Diclofenac enthalten. Selbstverständlich können diese Bereiche einander aber auch so zugeordnet werden, daß jeweils der größte allgemeine Bereich von Flupirtin dem größten allgemeinen Bereich des Antiphlogistikums zugeordnet wird (zum Beispiel Kombination aus 10 - 300 mg Flupirtin und 3 - 50 mg Diclofenac), der Vorzugsbereich von Flupirtin dem Vorzugsbereich des jeweiligen Antiphlogistikums und der "insbesondere-Bereich" von Flupirtin dem "insbesondere-Bereich" des jeweiligen Antiphlogistikums.

- Die in den vorangegangenen Seiten angegebenen Dosen und Gewichtsteile, die sich auf die Anwendung am Menschen beziehen, sind jeweils bezogen auf die freien Basen, beziehungsweise freien Säuren -

Die akute Toxizität der erfindungsgemäßen Kombinationen an der Maus (ausgedrückt durch die LD50 mg/kg; Methode: Litchfield und Wilcoxon, J. Pharmacol. Exper. Ther. 95: 99, 1949) liegt beispielsweise für die Kombination mit Flupirtin (Maleat) und Diclofenac (1 : 1) bei oraler Applikation bei 504 mg/kg beziehungsweise oberhalb von 471 mg/kg Körpergewicht. Die LD50 von Diclofenac allein ist zum Beispiel erheblich niedriger, nämlich 172 mg/kg. An der gleichen Versuchstierspecies werden hier weitere Beispiele angeführt:
Für die Kombination mit Flupirtin (Maleat) und Indometacin (1 : 1) bei oraler Applikation liegt die LD50 bei 633 mg/kg Körpergewicht beziehungsweise oberhalb von 577 mg/kg Körpergewicht. (LD50 von Indometacin allein: 21 mg/kg). Für die Kombination mit Flupirtin (Maleat) und Naproxen (1 : 1) bei oraler Applikation liegt die LD50 bei 613 mg/kg Körpergewicht beziehungsweise oberhalb von 605 mg/kg Körpergewicht.
Für die Kombination mit Flupirtin (Maleat) und Piroxicam (1 : 1) bei oraler Applikation liegt die LD50 bei 793 mg/kg Körpergewicht beziehungsweise oberhalb von 731 mg/kg Körpergewicht (LD50 von Piroxicam allein: 350 mg/kg).
Für die Kombination mit Flupirtin (Maleat) und Sulindac (1 : 1) bei oraler Applikation liegt die LD50 bei 550 mg/kg Körpergewicht beziehungsweise oberhalb von 505 mg/kg Körpergewicht (LD50 von Sulindac allein: 507 mg/kg).
Für die Kombination mit Flupirtin (Maleat) und Acetylsalicylsäure (ASS) (1 : 13,9) bei oraler Applikation liegt die LD50 bei 1942 mg/kg, bezogen auf Flupirtin, beziehungsweise bei 1679 mg/kg Körpergewicht, bezogen auf ASS (LD50 von ASS allein: 815 mg/kg).
Für die Kombination mit Flupirtin (Maleat) und Isoxicam (1 : 1) bei oraler Applikation liegt die LD50 bei 727 mg/kg Körpergewicht beziehungsweise oberhalb von 686 mg/kg Körpergewicht.

Von besonderer Bedeutung ist hierbei, daß die gastrointestinale Nebenwirkungsrate bei den erfindungsgemäßen Kombinationen von Flupirtin mit den nicht-steroidalen Antiphlogistika überraschend erniedrigt wird. So wurde zum Beispiel an der Albino-Ratte (Sprague-Dawley) kein ulzerogener Effekt (Magenschleimhauterosion) am Magen nach einmaliger per os-Verabreichung der Kombination von 30 mg/kg Flupirtin und 24 mg/kg Diclofenac 24 Stunden nach der Verabreichung festgestellt, während nach einer Verabreichung von 24 mg/kg Diclofenac allein (per os) nach 24 Stunden bei den Tieren Magenschleimhauterosionen auftraten. Ebenso können an dem gleichen Tiermodell beispielsweise nach einmaliger Verabreichung der Kombination von 30 mg/kg per os Flupirtin und 0,1 mg/kg per os Indometacin keine Magenulzera innerhalb von 24 Stunden ausgelöst werden.
Aus der folgenden Tabelle geht hervor, daß beispielsweise häufig die LD50 des nicht-steroidalen Antiphlogistikums durch die erfindungsgemäße Kombination erhöht wird, das heißt auch die Verträglichkeit des Antiphlogistikums wird durch die erfindungsgemäße Kombination erhöht. Dies gilt insbesondere für solche Antiphlogistika, die eine niedrige LD50 haben, das heißt schwer verträglich sind, wie zum Beispiel Indometacin, Diclofenac.

LD50-Werte für das nicht-steroidale Antiphlogistikum in der Kombination. Tier: NMRI-Maus, Applikation: per os
Methode: Litchfield; J. T., Wilcoxon, F.; J.Pharmacol. Exper.Ther. 95 : 99, 1949

| Wirkstoff-Kombiation Verhältnis von Flupirtin (Maleat) zum Antiphlogistikum (Gewichtsteile) | LD50-Werte des nicht-steroidalen Antiphlogistikums in der Kombination |
|---|---|

| | |
|---|---|
| Diclofenac, allein | 172 mg/kg (Arzneim.Forsch. 33, 1555, 83) |
| Flupirtin (Maleat) + Diclofenac 1 : 1 | 252 mg/kg |
| Flupirtin (Maleat) + Diclofenac 1 : 0,82 | 258 mg/kg |
| Indometacin, allein | 21 mg/kg (Arzneim.Forsch. 33, 1555, 83) |
| Flupirtin (Maleat) + Indometacin 1 : 1 | 316 mg/kg |
| Flupirtin (Maleat) + Indometacin 1 : 0,63 | 242 mg/kg |
| Pirocixam, allein | 350 mg/kg (Basic Pharmacology and Therapeutics 8,4639,80) |
| Flupirtin (Maleat) + Pirocixam 1 : 1 | 396 mg/kg |
| Acetylsalicylsäure, allein | 815 mg/kg (Toxicology and Applied Pharmacology 23, 537, 72) |
| Flupirtin (Maleat) + Acetylsalicylsäure 1 : 13,93 | 1811 mg/kg |

Die erfindungsgemäße Kombination ist zur Herstellung pharmazeutischer Zusammensetzungen und Zubereitungen geeignet. Die pharmazeutischen Zusammensetzungen beziehungsweise Arzneimittel enthalten als Wirkstoff Flupirtin sowie nicht-steroidale Antiphlogistika, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 u.ff., H.v.Czetsch-Lindenwald, Hilfsstoffe für Pharmazie und angrenzende Gebiete; Pharm. Ind., Heft 2, 1961, Seite 72 u.ff.; Dr. H. P. Fiedler, Lexikon der Hilfsstoffe für Pharmazie, Kosmetik und angrenzende Gebiete, 2. Auflage, Editio Cantor, Aulendorf in Württemberg 1981.

Beispiele hierfür sind Gelatine, natürliche Zucker wie Rohrzucker oder Milchzucker, Lecithin, Pektin, Stärke (zum Beispiel Maisstärke) sowie Stärkederivate, Cyclodextrine und Cyclodextrinderivate, Polyvinylpyrrolidon, Gelatine, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind (zum Beispiel Methylcellulose, Hydroxypropylmethylcellulose, Hydroxypropylmethylcellulosephthalat), Stearate, Magnesium- und Calciumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnußöl, Rhizinusöl, Olivenöl, Sesamöl, Baumwollsaatöl, Maisöl, Weizenkeimöl, Sonnenblumensamenöl, Kabeljau-Leberöl, Mono-, Di- und Triglyceride von gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen und Essigsäure, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden wie zum Beispiel: Polyacrylsäureester, Celluloseether und ähnliche.

Zur Herstellung von Lösungen und Suspensionen kommen beispielsweise Wasser oder physiologisch verträgliche organische Lösungsmittel in Frage, wie zum Beispiel Ethanol 1,2-Propylenglykol, Polyglykole und deren Derivate, Dimethylsulfoxyd, Fettalkohole, Triglyceride, Partialester des Glycerins, Paraffine und ähnliche. Für injizierbare Lösungen oder Suspensionen kommen zum Beispiel nicht-toxische parenteral verträgliche Verdünnungsmittel oder Lösungsmittel in Frage, wie zum Beispiel: Wasser, 1,3-Butandiol, Ethanol, 1,2-Propylenglykol, Polyglykole in Mischung mit Wasser, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.

Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen die Ölsäurereste enthalten) mit Ethylenoxyd erhalten werden (zum Beispiel 40 Mol Ethylenoxyd pro Mol Glycerid).

Beispiele für Oleotriglyceride sind Olivenöl, Erdnußöl, Rhizinusöl, Sesamöl, Baumwollsaatöl, Maisöl. Siehe auch Dr. H.P. Fiedler "Lexikon der Hilfsstoffe für Pharmazie, Kosmetik und angrenzende Gebiete" 1971, S. 191 - 195. Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) und dergleichen möglich. Gegebenenfalls ist zur Stabilisierung der Wirkstoffmoleküle mit physiologisch verträglichen Säuren oder Puffern auf einen pH-Bereich von ca. 2 bis 8 einzustellen. Im allgemeinen wird ein möglichst neutraler bis schwach saurer (bis pH 5) pH-Wert bevorzugt.

Als Antioxydantien kommen beispielsweise Natriummetabisulfit, Ascorbinsäure, Gallussäure, Gallussäurealkylester, Butylhydroxyanisol, Nordihydroguajaretsäure, Tocopherole sowie Tocopherole + Synergisten (Stoffe, die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgerät) gut vermischt, wobei im allgemeinen bei Temperaturen zwischen 20 und 80°C, vorzugsweise 20 bis 50°C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

**Kurze Beschreibung der in der Anmeldung erwähnten pharmakologischen Testmethoden**

Randall-Selitto-Test (Entzündungsschmerz an der Ratte):

In Anlehnung an die Methode von Randall und Selitto (L.O. Randall, J.J. Selitto, Arch. int. Pharmacodyn., Band 111, Seite 409 - 418, 1957) erhalten Ratten 0,1 ml einer 20 %-igen (in entmineralisiertem Wasser) Brauerhefesuspension in die rechte Hinterpfote subplantar injiziert. 2 1/2 Stunden danach werden die Prüfsubstanzen verabreicht und 30 Minuten später die Schmerzschwelle mit einem Algesiemeter als Druck (in Gramm) auf die entzündete Pfote gemessen (Geräte von Ugo Basile, Mailand, Italien). Als Kriterium gilt die Abwehrreaktion der Tiere, die Pfote wegzuziehen und/oder sich aus dem Griff des Experimentators zu befreien. Die Substanzwirkung wird aus der Erhöhung der Schmerzschwelle gegenüber einer unbehandelten Kontrollgruppe ermittelt. Der Versuchsablauf unterscheidet sich von der Originalmethode dadurch, daß die Substanzen erst 2 1/2 Stunden nach der Ödemsetzung und nicht mit dieser gleichzeitig verabreicht werden. Auf diese Weise soll verhindert werden, daß die Ödementwicklung durch eine mögliche antiphlogistische Wirkung gehemmt und die Analgesie überlagert beziehungsweise vorgetäuscht wird.

Bestimmt wird die ED50 mittels der Methode der linearen Regression. Die ED50 ist die Dosis in mg/kg, bei der rechnerisch eine 50 %-ige analgetische Wirkung vorliegt.

**Essigsäure-Test (Writhing-Test) an der Maus:**

Methode:

Im Essigsäure-Test nach Koster et al (Fed. Proc., Band 18, 1959, Seite 412) wird der Schmerzreiz durch eine intraperitoneale Injektion 1 %-iger Essigsäure ausgelöst. Die Schmerzreaktion äußert sich als ein charakteristisches Strecken der Tiere ("writhing syndrom"), das in unregelmäßigen Zeitabständen längere Zeit nach der Essigsäure-Injektion anhält. Die dosisabhängige Hemmung der Häufigkeit der Streckbewegung gegenüber einer unbehandelten Kontrollgruppe wird als analgetische Wirkung in Prozent ausgedrückt. Die Auswertung erfolgt durch Bestimmung der ED50 (Methode der linearen Regression). Die ED50 ist die Dosis in mg/kg, bei der rechnerisch eine 50 %-ige Hemmung des "Writhing syndroms" vorliegt.

Der Essigsäure-Test zeichnet sich dadurch aus, daß nicht nur die Wirkung von starken, zentral wirksamen Analgetika, sondern auch von überwiegend peripher wirksamen Analgetika-Antipyretika und antiphlogistisch wirksamen Pharmaka wie Phenylbutazon, Indometacin und andere nachweisbar ist. Damit weist die Wirkung in dieser Versuchsanordnung auf eine periphere Komponente der Analgesie hin.

**Hot-Plate-Test:**

In Anlehnung an die Methode von Janssen (P.A.J. Janssen and A.H. Jageneau, J. Pharm. Pharmacol., Band 9, 1957, Seite 381 - 400) werden Mäuse auf eine 55,5°C heiße Platte gesetzt. Gewertet wird die Reaktionszeit bis zum "Belecken" der Pfoten. Die analgetische Wirkung der Substanzen wird als Verlängerung der Reaktionszeit (in Sekunden) gegenüber einer nur mit Vehikel behandelten Kontrollgruppe in Prozent ausgedrückt.

Als ED50 gilt die Dosis in mg/kg, bei der rechnerisch eine 50 %-ige analgetische Wirkung vorliegt.

**Carrageenin-Ödem-Test auf antiphlogistische Wirkung:**

In Anlehnung und Modifikation der Methode von Mörsdorf et al (Arch. int. Pharmacodyn. 192, 111 - 127 (1971)) erhielten männliche Ratten 0,1 ml einer 1 %-igen Carrageenin-Lösung* subplantar in die rechte Hinterpfote injiziert. Eine Stunde und vier Stunden danach wird das Pfotenvolumen ermittelt. Zur Volumenmessung wird ein Volumen-Meter (Firma Rhema-Labortechnik, 6238 Hofheim, Typ 2060) verwendet. Unmittelbar nach der ersten Messung wird die Testsubstanz mittels Magenschlundsonde verabreicht. Berechnet wird die Zunahme der Pfotenschwellung der zweiten Messung gegenüber der ersten Messung in absoluten Werten (ml). Die Auswertung umfaßt die Ermittlung der prozentualen Mittelwerte der Differenzen der Ödemvolumina eine und vier Stunden nach Entzündungsauslösung. Die ödemhemmende Wirkung wird aus den durch Wasserverdrängung gemessenen Ödemvolumina im Vergleich zu den Ödemvolumina der unbehandelten, entzündeten Kontrollgruppe in Prozent berechnet.

Als $ED_{50}$ gilt die Dosis in mg/kg, bei der rechnerisch eine 50 %-ige Ödemhemmung vorliegt.

* Carrageenin, Typ IV, Sigma C-3889

**Bradykinin-Ödem-Test auf antiphlogistische Wirkung:**

Als einzige Abänderung im Vergleich zum Carrageenin-Ödem-Test wird anstelle des Carrageenins das Bradykinin (Sigma B 3259) 0,01 mg/Tier in 0,1 ml einer 0,9 %-igen NaCl-Lösung subplantar injiziert.

**Adjuvans-Arthritis-Test an der Ratte auf antiphlogistische Wirkung:**

In Anlehnung an Newbould (Brit. J. Pharmacol., Band 21, 1963, Seite 127 - 136) wird die Adjuvans-Arthritis durch Mycobacterium butyricum ausgelöst, welches in die rechte Hinterpfote der Ratten subplantar injiziert wird. Die abgetöteten Mycobacterien butyrica werden für die Injektion in Freund'schem Adjuvans (flüssiges Paraffin) aufgeschwemmt.

Bei den Kontrolltieren (Tiere, die keine Testsubstanzen erhalten) zeigt sich hierbei schon nach 24 Stunden ein meßbares Ödem an der injizierten Pfote, das etwa 10 bis 12 Tage lang fast unverändert groß bleibt (akute Phase der Arthritis). Danach setzt an der injizierten Pfote eine weitere Volumenzunahme des Ödems ein (sekundäre Phase der Arthritis = chronische Entzündung), gemischt mit Resten der akuten Entzündung der ersten 10 Tage. Gleichzeitig mit diesem zweiten Schub an der injizierten Pfote setzt eine Ödembildung auch an

der mit dem Bakterium nicht injizierten Pfote ein (chronisch-immunologische Entzündung).

Die Tiere, an denen die Testsubstanzen bei prophylaktischer Applikation geprüft werden, erhalten bereits 2 Tage vor Auslösung der Entzündung die Testsubstanz und die Tiere, an denen die Testsubstanzen bei therapeutischer Applikation geprüft werden, erhalten erst 8 Tage nach Auslösung der Entzündung einmal täglich per os die Testsubstanz (das heißt, die Kombination Flupirtin + Antiphlogistikum, zum Beispiel im Verhältnis 10 Gewichtsteile Flupirtin zu 1 oder 3 Gewichtsteile Antiphlogistikum). Am dritten beziehungsweise neunten Tag (je nach Versuchsaufbau: Prophylaxe oder Therapie) wird dann die Arthritis durch Injektion ausgelöst, wobei die Testsubstanzen in gleicher Weise einmal täglich per os während der ganzen Versuchsdauer verabreicht werden. Die Kombination Flupirtin - Antiphlogistikum wird in Methylcellulose verabreicht. Die Gesamtversuchsdauer mit prophylaktischer Applikation der Prüfsubstanzen beträgt 14 Tage und die mit therapeutischer Applikation 35 Tage. Die antiphlogistische Wirkung wird aus den durch Wasserverdrängung gemessenen Ödemvolumina im Vergleich zu den Ödemvolumina der unbehandelten Kontrollgruppe in Prozent berechnet sowie durch Bestimmung der Maximumsgröße aller Differenzen zum Tag 0*. Parallel wird als Standardpräparat Indometacin mitgeprüft. In der Auswertung ist auch die Bestimmung der $ED_{50}$ (Dosis, die 50 % Hemmung der Volumenzunahme des Ödems hervorruft) nach der Methode der linearen Regression enthalten.

* Bestimmung der Maximumsgröße aller Differenzen zum Tag 0 bedeutet folgendes:

In dem Versuchszeitraum wird einmal täglich bei den einzelnen Tieren die Pfotenschwellung (das heißt das Pfotenvolumen) gemessen (durch Eintauchen der Pfote in Wasser) und die Differenz jeweils zu dem Pfotenvolumen am Tage 0 bestimmt. Hieraus ergibt sich unmittelbar der Maximalwert der Schwellung während des Versuchs. Man errechnet dann den Mittelwert der Maximal-Schwellung und die dazugehörige Standardabweichung.

Prüfung auf ulcerogene Wirkung in Anlehnung an die Methode von Jahn und Adrian (Arzneimittelforschung, Band 19, 1969, Seite 36 - 52):

Die Versuchstiere sind männliche Albino-Ratten, die unter den üblichen Bedingungen bei 21 - 22° C gehalten werden. Nach 48-stündiger Nahrungskarenz werden die Testsubstanzen in ansteigenden Dosen peroral verabreicht. 24 Stunden danach werden die Tiere getötet und die Mägen auf ulcerogene Veränderungen nach der Methode von Münchow, Arzneimittelforschung, Band 4, 1954, Seite 341 - 344 untersucht. (Die Größe der gebildeten Ulcera wird gemessen und nach Münchow klassifiziert.)

**Beispiel 1**

Kapseln mit 150 mg Flupirtinmaleat und 20 mg Diclofenac-Natrium:

300 g Flupirtinmaleat werden mit 40 g Diclofenac-Natrium gemischt und anchließend mit einer Lösung aus 8 g Kollidon VA 64® (BASF)* in 300 ml Wasser in üblicher Weise granuliert. Das getrocknete Granulat wird durch ein Sieb der Maschenweite 1 mm gegeben und anschließend mit 1 g Magnesiumstearat und 1 g Hochdispersem Siliciumdioxid (Aerosil 200/Degussa) vermischt.

Die Mischung wird zu jeweils 175 mg in Hartgelatine-Kapseln der Größe 1 gefüllt.

Eine Kapsel enthält 150 mg Flupirtinmaleat und 20 mg Diclofenac-Natrium.

In analoger Weise können Kapseln mit 100 mg Flupirtinmaleat und 25 mg Diclofenac-Natrium hergestellt werden.

* Kollidon VA 64® ist ein Vinylpyrrolidon-Vinylacetat-Copolymerisat 60 : 40.

**Beispiel 2**

Tabletten mit 150 mg Flupirtinmaleat und 20 mg Diclofenac-Natrium:

300 g Flupirtinmaleat werden mit 40 g Diclofenac-Natrium gemischt und die Mischung mit einem Schleim aus 20 g Maisstärke in 370 g Wasser in üblicher Weise granuliert. Nach dem Trocknen wird das Granulat durch ein Sieb der Maschenweite 0,8 mm gegeben und anschließend mit 300 g Mikrokristalliner Cellulose, 52 g Modifizierter Stärke (Starch 1500/Colorcon®)*, 7 g Magnesiumstearat sowie 1 g Hochdispersem Siliciumdioxid (Aerosil 200®/Degussa) gemischt. Die Mischung wird zu Oblong-Tabletten vom Gewicht 360 mg, einer Länge von 16 mm und einer Breite von 7 mm verpreßt.

Anschließend können die Tabletten gegebenenfalls in der üblichen Weise mit einem magensaftresistenten beziehungsweise magensaftpermeablen oder -löslichen Filmüberzug versehen werden.

Eine Tablette enthält 150 mg Flupirtinmaleat sowie 20 mg Diclofenac-Natrium.

* Bei der modifizierten Stärke handelt es sich um eine freifließende und teilweise kaltwasserlösliche Maisstärke; diese Modifikation erfolgt durch rein physikalische Maßnahmen.

**Beispiel 3**

Kapseln mit 40 mg beziehungsweise 20 mg Flupirtinmaleat und 10 mg beziehungsweise 5 mg Diclofenac-Natrium:

80 g Flupirtinmaleat werden mit 20 g Diclofenac-Natrium und 150 g Calciumhydrogenphosphat gemischt und anschließend mit einer Lösung von 3 g Kollidon VA 64® (BASF) in 115 ml Wasser in üblicher Weise granuliert. Das getrocknete Granulat wird durch ein Sieb der Maschenweite 0,8 mm gegeben und anschließend mit 4 g Magnesiumstearat, 1 g Hochdispersem Siliciumdioxid und 42 g Modifizierter Stärke (Starch 1500/Colorcon®) gemischt.

Die Mischung wird zu jeweils 150 mg in Hartgelatine-Kapseln der Größe 3 gefüllt.

Eine Kapsel enthält 40 mg Flupirtinmaleat und 10 mg Diclofenac-Natrium.

In ähnlicher Weise lassen sich Kapseln mit 20 mg Flupirtinmaleat und 5 mg Diclofenac-Natrium herstellen, indem eine Mischung von 40 g Flupirtinmaleat, 10 g Diclofenac-Natrium und 200 g Calciumhydrogenphosphat mit einer Lösung von 3 g Kollidon VA 64® (BASF) in 75 ml Wasser granuliert und in der angegebenen Weise weiterverarbeitet wird.

**Beispiel 4**

Tabletten mit 40 mg beziehungsweise 20 mg Flupirtinmaleat und 10 mg beziehungsweise 5 mg Diclofenac-Natrium:

280 g Flupirtinmaleat werden mit 70 g Diclofenac-Natrium gemischt und die Mischung mit einem Schleim aus 21 g Maisstärke in 400 g Wasser in üblicher Weise granuliert. Nach dem Trocknen wird das Granulat durch ein Sieb der Maschenweite 0,8 mm gegeben und anschließend mit 280 g Mikrokristalliner Cellulose, 40,6 g Modifizierter Stärke (Starch 1500/Colorcon®), 1,4 g Hochdispersem Siliciumdioxid und 7 g Magnesiumstearat gemischt. Die Mischung wird zu bikonvexen Tabletten vom Gewicht 100 mg, einem Durchmesser von 6 mm und einem Wölbungsradius von 4 mm gepreßt.

Anschließend können die Tabletten gegebenenfalls in der üblichen Weise mit einem magensaftresistenten beziehungsweise magensaftpermeablen beziehungsweise -löslichen Filmüberzug versehen werden.

Eine Tablette enthält 40 mg Flupirtinmaleat und 10 mg Diclofenac-Natrium.

In ähnlicher Weise können Tabletten mit 20 mg Flupirtinmaleat und 5 mg Diclofenac-Natrium hergestellt werden, indem 140 g Flupirtinmaleat mit 35 g Diclofenac-Natrium und 175 g Calciumhydrogenphosphat gemischt und die Mischung mit einem Schleim aus 21 g Maisstärke in 280 g Wasser granuliert und nach dem Trocknen wie oben angegeben weiterverarbeitet wird.

**Beispiel 5**

Suppositorien mit 150 mg Flupirtinmaleat und 20 mg Diclofenac-Natrium:

75 g Flupirtinmaleat und 10 g Diclofenac-Natrium werden in 950 g geschmolzenem Hartfett (siehe Europäisches Arzneibuch, Band III)* suspendiert. Nach Homogenisierung wird die Suspension in üblicher Weise in Hohlzellen von 2,3 ml ausgegossen und abgekühlt.

Ein Suppositorium vom Gewicht 2,07 g enthält 150 mg Flupirtinmaleat und 20 mg Diclofenac-Natrium.

In analoger Weise können Suppositorien mit 150 mg Flupirtinmaleat und 25 mg Diclofenac-Natrium hergestellt werden (die Menge an geschmolzenem Hartfett ist dann entsprechend zu verringern).

* Hartfett ist ein Gemisch von Mono-, Di- und Triglyceriden der gesättigten Fettsäuren von $C_{10}H_{20}O_2$ bis $C_{18}H_{36}O_2$.

**Beispiel 6**

Suppositorien mit 40 mg beziehungsweise 20 mg Flupirtinmaleat und 10 mg beziehungsweise 5 mg Diclofenac-Natrium:

20 g Flupirtinmaleat und 5 g Diclofenac-Natrium werden in 995 g geschmolzenem Hartfett suspendiert. Nach Homogenisierung wird die Suspension in üblicher Weise in Hohlzellen von 2,3 ml ausgegossen und abgekühlt.

Ein Suppositorium vom Gewicht 2,04 g enthält 40 mg Flupirtinmaleat und 10 mg Diclofenac-Natrium.

In ähnlicher Weise können Suppositorien mit 20 mg Flupirtinmaleat und 5 mg Diclofenac-Natrium hergestellt werden, indem statt 995 g geschmolzenem Hartfett 2015 g geschmolzenes Hartfett eingesetzt, ansonsten aber in gleicher Weise gearbeitet wird.

**Beispiel 7**

Kapseln mit 40 mg beziehungsweise 20 mg Flupirtinmaleat und 6 mg beziehungsweise 3 mg Piroxicam:

80 g Flupirtinmaleat werden mit 12 g Piroxicam und 158 g Calciumhydrogenphosphat gemischt und die Mischung mit einer Lösung von 3 g Kollidon VA 64® (BASF) in 120 ml Wasser in üblicher Weise granuliert. Das getrocknete Granulat wird durch ein Sieb der Maschenweite 0,8 mm gegeben und anschließend mit 4 g Magnesiumstearat, 1 g Hochdispersem Siliciumdioxid und 42 g Modifizierter Stärke (Starch 1500/Colorcon®) gemischt.

Die Mischung wird zu jeweils 150 mg in Hartgelatine-Kapseln der Größe 3 gefüllt.

Eine Kapsel enthält 40 mg Flupirtinmaleat und 6 mg Piroxicam.

In ähnlicher Weise können Kapseln mit 20 mg Flupirtinmaleat und 3 mg Piroxicam hergestellt werden, indem 40 g Flupirtinmaleat mit 6 g Piroxicam und 204 g Calciumhydrogenphosphat gemischt, die Mischung mit einer Lösung von 3 g Kollidon VA 64® (BASF) in 75 ml Wasser granuliert und das Granulat nach dem Trocknen in der oben angegebenen Weise weiterverarbeitet wird.

**Beispiel 8**

Suppositorien mit 40 mg beziehungsweise 20 mg Flupirtinmaleat und 6 mg beziehungsweise 3 mg Piroxicam:

20 g Flupirtinmaleat und 3 g Piroxicam werden in 997 g geschmolzenem Hartfett suspendiert. Nach Homogenisierung wird die Suspension in üblicher Weise in Hohlzellen von 2,3 ml ausgegossen und abgekühlt.

Ein Suppositorium vom Gewicht 2,04 g enthält 40 mg Flupirtinmaleat und 6 mg Piroxicam.

In ähnlicher Weise können Suppositorien mit 20 mg Flupirtinmaleat und 3 mg Piroxicam hergestellt werden, indem statt 997 g geschmolzenem Hartfett 2017 g geschmolzenes Hartfett eingesetzt, ansonsten aber in gleicher Weise gearbeitet wird.

**Patentansprüche** für die Vertragsstaten: BE, CH, DE, FR, GB, IT, LI, LU, NL, SE

1. Synergistisch wirksames Arzneimittel, mit analgetischer und antiphlogistischer Aktivität enthaltend als Wirkstoff Flupirtin und Acetylsalicylsäure oder ein nicht-steroidales Antiphlogistikum, welches ein Arylessigsäure- oder Arylpropionsäurederivat oder ein Oxicam ist oder ein Salz dieser Verbindungen mit physiologisch unbedenklichen Säuren beziehungsweise physiologisch unbedenklichen Metallen, wobei die Kombination in der Dosierungseinheit 10 bis 1800 mg Flupirtin und 1 bis 1600 mg des Antiphlogistikums enthält und die Menge des nicht-steroidalen Antiphlogistikums 0,05 bis 120 Gewichtsteile bezogen auf einen Gewichtsteil Flupirtin beträgt.

2. Arzneimittel nach Anspruch 1,
dadurch gekennzeichnet, daß die Kombination 10 - 1200 mg, vorzugsweise 15 - 900 mg Flupirtin enthält und 2 - 500 mg Acetylsalicylsäure beziehungsweise nichtsteroidales Antiphlogistikum.

3. Arzneimittel nach Anspruch 1,
dadurch gekennzeichnet, daß die Kombination 50 - 600 mg, vorzugsweise 50 - 400 mg Flupirtin enthält und 5 - 300 mg Acetylsalicylsäure beziehungsweise nichtsteroidales Antiphlogistikum.

**Patentansprüche** für den Vertragsstaat: AT

1. Verfahren zur Herstellung eines synergistisch wirksames Arzneimittels mit analgetischer und Antiphlogistischer Aktivität, dadurch gekennzeichnet, daß man den Wirkstoff Flupirtin und Acetylsalicylsäure oder Flupirtin und ein nicht-steroidales Antiphlogistikum, welches ein Arylessigsäure- oder Arylpropionsäurederivat oder ein Oxicam ist oder ein Salz dieser Verbindungen mit physiologisch unbedenklichen Säuren beziehungsweise physiologisch unbedenklichen Metallen gegebenenfalls mit weiteren üblichen Träger- und/oder Verdünnungs- beziehungsweise Hilfsstoffen zu pharmazeutischen Zubereitungen formuliert, wobei die Menge Acetylsalicylsäure oder nicht-steroidales Antiphlogistikum 0,05 bis 120 Gewichtsteile bezogen auf einen Gewichtsteil Flupirtin beträgt und wobei 10 bis 1800 mg Flupirtin und 1 bis 1600 mg Acetylsalicylsäure oder nicht-steroidales Antiphlogistikum zu einer Dosierungseinheit formuliert werden.

2. Verwendung von Flupirtin und Acetylsalicylsäure oder Flupirtin und einem nicht-steroidalen Antiphlogistikum, welches ein Arylessigsäure- oder Arylpropionsäurederivat oder ein Oxicam ist oder einem Salz dieser Verbindungen mit physiologisch unbedenklichen Säuren beziehungsweise physiologisch unbedenklichen Metallen, wobei die Kombination in der Dosierungseinheit 10 bis 1800 mg Flupirtin und 1 bis 1600 mg Acetylsalicylsäure oder nicht-steroidales Antiphlogistikum gemäß Anspruch 1 enthält, und die Menge Acetylsalicylsäure oder nicht-steroidales Antiphlogistikum 0,05 bis 120 Gewichtsteile, bezogen auf einen Gewichtsteil Flupirtin, beträgt, zur Herstellung eines analgetisch wirksamen Arzneimittels.

3. Verfahren zur Herstellung eines (analgetisch wirksamen) Mittels nach Anspruch 1 und 2, dadurch gekennzeichnet, daß man 1 Gewichtsteil Flupirtin und 0,05 - 120 Gewichtsteile Acetylsalicylsäure oder 1 Gewichtsteil Flupirtin und 0,05 - 120 Gewichtsteile eines nicht-steroidalen Antiphlogistikums, welches ein Arylessigsäure- oder Arylpropionsäurederivat oder ein Oxicam ist oder deren Salze mit physiologisch unbedenklichen Säuren beziehungsweise physiologisch unbedenklichen Metallen zusammen mit üblichen Träger- und/oder Verdünnungs- beziehungsweise Hilfsstoffen bei Temperaturen zwischen 20 und 80°C vermischt beziehungsweise homogenisiert, die so erhaltene Mischung zur Herstellung von Zubereitungen, die in der Dosierungseinheit 1 bis 1800 mg Flupirtin und 1 bis 1600 mg Acetylsalicylsäure oder nicht-steroidales Antiphlogistikum enthalten, beispielsweise zur Herstellung von Suppositorien in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

4. Verfahren zur Herstellung eines Mittels nach Anspruch 1, dadurch gekennzeichnet, daß man 1 Gewichtsteil Flupirtin und 0.05 - 120 Gewichtsteile Acetylsalicylsäure oder eines nicht-steroidalen Antiphlogistikums gemäß Anspruch 1 oder deren Salze mit physiologisch unbedenklichen Säuren beziehungsweise physiologisch unbedenklichen Metallen, gegebenenfalls mit mindestens einem der Hilfsstoffe Stärke, mikrokristalline Cellulose, Calciumhydrogenphosphat und modifizierte Stärke vermischt, mit einer wässrigen Gelatinelösung oder Stärkelösung oder einem wässrigen Vinylpyrrolidon-Vinylacetat Copolymerisat 60 : 40 granuliert und das erhaltene Granulat mit Magnesiumstearat und hochdispersem Siliziumdioxid sowie gegebenenfalls auch Stärke und/oder Cellulose vermischt und zu Tabletten verpresst oder in Kapseln abfüllt.

5. Verfahren zur Herstellung eines Mittels nach Anspruch 1, dadurch gekennzeichnet, daß man 1 Gewichtsteil Flupirtin und 0.05 - 120 Gewichtsteile Acetylsalicylsäure oder eines nicht-steroidalen Antiphlogistikums gemäß Anspruch 1 oder deren Salze mit physiologisch unbedenklichen Säuren beziehungsweise physiologisch unbedenklichen Metallen, gegebenenfalls nach Zusatz von Lecithin in geschmolzenem Hartfett suspendiert und homogenisiert und anschließend die Mischung in Hohlzellen ausgießt.

**Claims** for the contracting states: BE, CH, DE, FR, GB, IT, LI, LU, NL, SE

1. A synergistic medicament having analgesic and antiphlogistic activity containing as active principle flupirtin and acetyl salicylic acid or a non-steroidal antiphlogistic, which is an arylacetic acid or arylpropionic acid derivative or an oxicam, or a salt of these compounds with physiologically acceptable acids or phsyiologically acceptable metals, the combination containing 10 to 1800 mg flupurtin and 1 to 1600 g of the antiphlogistic per dosage unit and the quantity of the nonsteroidal antiphlogistic being from 0.05 to 120 parts by weight per part by weight flupirtin.

2. A medicament as claimed in claim 1, characterized in that the combination contains 10 to 1200 mg and preferably 10 to 900 mg flupurtin and 2 to 500 mg acetyl salicylic acid or non-steroidal antiphlogistic.

3. A medicament as claimed in claim 1, characterized in that the combination contains 50 to 600 mg and preferably 50 to 400 mg flupirtin and 5 to 300 mg acetyl salicylic acid or non-steroidal antiphlogistic.

**Claims** for the contracting state: AT

1. A process for the preparation of a synergistic medicament having analgesic and antiphlogistic activity, characterized in that the active principle flupirtin and acetyl salicylic acid or flupirtin and a non-steroidal antiphlogistic, which is an aryl acetic acid or aryl propionic acid derivative or an oxicam, or a salt of these compounds with physiologically acceptable acids or physiologically acceptable metals, is formulated, optionally with other standard excipients and/or diluents or auxiliaries, to form pharmaceutical preparations, the quantity of acetyl salicylic acid or non-steroidal antiphlogistic comprising 0.05 to 120 parts by weight per part by weight flupirtin and 10 to 1800 mg flupirtin and 1 to 1600 mg acetyl salicylic acid or non-steroidal antiphlogistic being formulated into a dosage unit.

2. The use of flupirtin and acetyl salicylic acid or flupirtin and a non-steroidal antiphlogistic, which is an aryl acetic acid or aryl propionic acid derivative or an oxicam, or a salt of these compounds with physiologically acceptable acids or physiologically acceptable metals, the combination containing 10 to 1800 mg flupirtin and 1 to 1600 mg acetyl salicylic acid or non-steroidal antiphlogistic according to claim 1 per dosage unit and the quantity of acetyl salicylic acid or non-steroidal antiphlogistic comprising 0.05 to 120 parts by weight per part by weight flupirtin, for the preparation of a medicament having analgesic activity.

3. A process for the preparation of an (analgesic) agent according to claims 1 and 2, characterized in that 1 part by weight flupirtin and 0.05 to 120 parts by weight acetyl salicylic acid or 1 part by weight flupirtin and 0.05 to 120 parts by weight of a non-steroidal antiphlogistic, which is an aryl acetic acid or acyl propionic acid derivative or an oxicam, or salts thereof with physiologically acceptable acids or physiologically acceptable metals are mixed or homogenized together with standard excipients and/or diluents or auxiliaries at

temperatures in the range from 20 to 80°C, and, to produce preparations containing 1 to 1800 mg flupirtin and 1 to 1600 mg acetyl salicylic acid or non-steroidal antiphlogistic per dosage unit, for example suppositories, the resulting mixture is poured out into hollow cells of appropriate size or is packed in capsules of appropriate size or is granulated and then compressed, optionally with addition of other standard auxiliaries, to form tablets.

4. A process for the preparation of an agent according to claim 1, characterized in that 1 part by weight flupirtin and 0.05 to 120 parts by weight acetyl salicylic acid or a non-steroidal antiphlogistic according to claim 1 or salts thereof with physiologically acceptable acids or physiologically acceptable metals, are optionally mixed with at least one of the auxiliaries starch, microcrystalline cellulose, calcium hydrogen phosphate and modified starch, the resulting mixture is granulated with an aqueous gelatin solution or starch solution and an aqueous vinyl pyrrolidone/vinyl acetate copolymer (60 : 40) and the granulate obtained is mixed with magnesium stearate and highly disperse silicon dioxide and, optionally, starch and/or cellulose and compressed to tablets or packed in capsules.

5. A process for the preparation of an agent according to claim 1, characterized in that 1 part by weight flupirtin and 0.05 to 120 parts by weight acetyl salicylic acid or a non-steroidal antiphlogistic according to claim 1 or salts thereof with physiologically acceptable acids or physiologically acceptable metals are suspended in molten hard fat, optionally after addition of lecithin, and homogenized and the mixture obtained is subsequently poured out into hollow cells.

**Revendications** pour les Etats contractants: BE, CH, DE, GB, IT, LI, LU, SE

1. Médicament à action synergique possédant une activité analgésique et antiphlogistique, contenant, en tant que substance active, de la flupirtine et de l'acide acétylsalicylique ou un antiphlogistique non stéroïdien qui est un dérivé d'acide arylacétique ou arylpropionique, un oxicam ou un sel de ces composés avec des acides physiologiquement acceptables ou des métaux physiologiquement acceptables, la combinaison contenant, dans l'unité posologique, 10 à 1800 mg de flupirtine et 1 à 1600 mg de l'antiphlogistique et la proportion de l'antiphlogistique non stéroïdien s'élevant à 0,05 - 120 parties en poids par rapport à 1 partie en poids de flupirtine.

2. Médicament selon la revendication 1, caractérisé en ce que la combinaison contient 10 - 1200 mg, de préférence 15 - 900 mg de flupirtine et 2 - 500 mg d'acide acétylsalicylique ou d'antiphlogistique non stéroïdien.

3. Médicament selon la revendication 1, caractérisé en ce que la combinaison contient 50 - 600 mg, de préférence 50 - 400 mg de flupirtine et 5 - 300 mg d'acide acétylsalicylique ou d'antiphlogistique non stéroïdien.

**Revendications** pour l'Etats contractant: AT

1. Procédé de préparation d'un médicament à action synergique possédant une activité analgésique et antiphlogistique, caractérisé en ce qu'on formule sous forme de préparations pharmaceutiques la substance active flupirtine et acide acétylsalicylique ou flupirtine et un antiphlogistique non stéroïdien, qui est un dérivé d'acide arylacétique ou arylpropionique ou un oxicam, ou un sel de ces composés avec des acides physiologiquement acceptables ou des métaux physiologiquement acceptables, éventuellement avec des excipients et/eu diluants ou adjuvants usuels supplémentaires, la proportion d'acide acétylsalicylique ou d'antiphlogistique non stéroïdien s'élevant à 0,05 - 120 parties en poids par rapport à 1 partie en poids de flupirtine, et 10 à 1800 mg de flupirtine et 1 à 1600 mg d'acide acétylsalicylique ou d'antiphlogistique non stéroïdien étant formulés en une unité posologique.

2. Utilisation, pour la préparation d'un médicament à action analgésique, de flupirtine et d'acide acétylsalicylique ou de flupirtine et d'un antiphlogistique non stéroïdien, qui est un dérivé d'acide arylacétique ou arylpropionique ou un oxicam, ou un sel de ces composés avec des acides physiologiquement acceptables ou des métaux physiologiquement acceptables, la combinaison contenant, par unité posologique, 10 à 1800 mg de flupirtine et 1 à 1600 mg d'acide acétylsalicylique ou d'antiphlogistique non stéroïdien selon la revendication 1, et la proportion d'acide acétylsalicylique ou d'antiphlogistique non stéroïdien s'élevant à 0,05 - 120 parties en poids par rapport à 1 partie en poids de flupirtine.

3. Procédé de préparation d'un agent (à action analgésique) selon la revendication 1 ou 2, caractérisé en ce qu'on mélange ou homogénéise, à une température comprise entre 20 et 80°C, 1 partie en poids de flupirtine et 0,05 - 120 parties en poids d'acide acétylsalicylique ou 1 partie en poids de flupirtine et 0,05 - 120 parties en poids d'un antiphlogistique non stéroïdien, qui est un dérivé d'acide arylacétique ou arylpropionique ou un oxicam, ou un sel de ceux-ci avec des acides physiologiquement acceptables ou des métaux physiologiquement acceptables, avec des excipients et/ou diluants ou adjuvants usuels et, pour la confection de préparations qui contiennent, par unité posologique, 1 à 1800 mg de flupirtine et 1 à 1600 mg d'acide acétylsalicylique ou d'antiphlogistique non stéroïdien, on verse le mélange ainsi obtenu dans des cellules creuses de taille appropriée, par exemple pour la confection de suppositoires, ou on le charge dans des capsules de taille appropriée ou on le granule, puis on le presse en comprimés, éventuellement avec addition d'adjuvants supplémentaires usuels.

4. Procédé de préparation d'un agent selon la revendication 1, caractérisé en ce qu'on mélange 1 partie en poids de flupirtine et 0,05 - 120 parties en poids d'acide acétylsalicylique ou d'un antiphlogistique non stéroïdien selon la revendication 1 ou des sels de ceux-ci avec des acides physiologiquement acceptables ou des métaux physiologiquement acceptables, éventuellement avec au moins l'un des adjuvants amidon, cellulose microcristalline, hydrogénophosphate de calcium et amidon modifié, on granule avec une solution aqueuse de gélatine ou d'amidon ou avec un copolymère vinylpyrrolidone/acétate de vinyle 60 : 40 aqueux, on mélange le granulé obtenu avec du stéarate de magnésium et du bioxyde de silicium fortement dispersé, ainsi éventuellement qu'avec de l'amidon et/ou de la cellulose, et on le presse en comprimés ou on en charge des capsules.

5. Procédé de préparation d'un agent selon la revendication 1, caractérisé en ce qu'on met en suspension et on homogénéise, dans une graisse dure fondue, éventuellement après addition de lécithine, 1 partie en poids de flupirtine et 0,05 - 120 parties en poids d'acide acétylsalicylique, d'un antiphlogistique non stéroïdien selon la revendication 1 ou de sels de ceux-ci avec des acides physiologiqueent acceptables ou des métaux physiologiquement acceptables, puis on verse le mélange dans des cellules creuses.